Europäisches Patentamt

European Patent Office

Office européen des brevets

(19)

(11) EP 0 789 307 A1

(12) **EUROPEAN PATENT APPLICATION**

(43) Date of publication:
13.08.1997 Bulletin 1997/33

(51) Int Cl.⁶: **G06F 17/00**

(21) Application number: 97300600.0

(22) Date of filing: 30.01.1997

(84) Designated Contracting States:
**AT BE CH DE DK ES FI FR GB GR IE IT LI LU MC NL PT SE**
Designated Extension States:
**SI**

(30) Priority: **02.02.1996 US 11772**

(71) Applicant: **SMITHKLINE BEECHAM CORPORATION**
**Philadelphia Pennsylvania 19103 (US)**

(72) Inventors:
• **Wong, Bruce**
**Philadelphia, Pennsylvania 19103 (US)**

• **Atkins, Dan,**
**Diversified Pharmaceuticals Services**
**South Edina, MN 55435 (US)**
• **Friedman, Felix,**
**Diversified Pharmaceuticals Serv.**
**South Edina, MN 55435 (US)**

(74) Representative: **Thompson, Clive Beresford et al**
**SmithKline Beecham plc**
**Corporate Intellectual Property,**
**Two New Horizons Court**
**Brentford, Middlesex TW8 9EP (GB)**

(54) **Method and system for identifying at risk patients diagnosed with depression**

(57)     A computer-implemented technique, including database processing, is used for identifying at risk exists in a claims database. The technique includes processing the patient information in the claims database to find and extract claims information for a group of depression patients. Next, using the extracted information, a set of events, relevant to depression, is defined. Next, the extracted information and set of events are processed to create event level information which is organized with respect to events rather than claims. A time window is defined for providing a timeframe from which to judge whether events should be considered in subsequent processing; and, a set of variables is defined as being potential predictors of adverse health outcomes. Subsequently, the event level information, using the time window and the set of variables, is processed to generate an analysis file. Statistical analysis, such as logistic regression, is performed on the analysis file to generate a prediction model where the prediction model is a function of a subset of the set of variables. Finally, the prediction model isand output at risk patients, diagnosed with depression, likely to have adverse health outcomes.

Claims Information

Store Raw Info In Database — 110

Pre-Process Raw Info — 112

**FIG. 1A**

Store in Research Database — 114

Extract CHF Patients — 116

Quality Check (optional) — 118

OK ? — N / Y

Convert Claims Level Data to Event Level Data — 120

Process Event Level Files into Analysis Files — 122 — timeframe / variables

Process Analysis File Using Stat Techinques — 124

Prediction Model

**Description**

BACKGROUND OF THE INVENTION

This invention relates to database processing techniques and, more particularly, it relates to identification of depression patients having a high risk of adverse health outcomes using various database processing techniques.

Depression is one of the most common treatable conditions affecting our society. In fact, depression occurs at levels comparable to angina and coronary artery disease. The point prevalence for major depression in western industrialized nations is 2.3 to 3.2% for men and 4.5 to 9.3% for women. The life time risk for depression is 7 to 12% for men and 20 to 25% for women. These statistics reflect the substantial burden depression has on society.

The economic burden of depression, however, is more difficult to quantify. Some estimates show that depression accounts for approximately 1/3 of the direct costs of all mental illnesses ($67 billion in 1990). Of the depression related costs, approximately 2/3 are related to direct medical expenditure. Although estimates of the economic cost of depression vary, they were conservatively estimated at $16.3 billion in 1980 of which approximately 2/3 were direct medical costs.

Depression is widely perceived as an essentially self-limiting condition, where a background of good functioning is punctuated by brief periods of illness and subsequent recovery. Over 50% of patients have recurrent episodes of depression. Treatment can then be viewed as being of an episodic nature with management of individual episodes.

The current Practice Guideline For Major Depressive Disorder in Adults published by the American Psychiatric Association (APA) in 1993 describe various means of diagnosing and treating depression and is herein incorporated by reference for its teachings about depression diagnosis and treatment. Other literature also exists, for example, literature published by the Agency of Health Care Policy Reasearch (AHCPR), which describes the illness, its symptoms and means of diagnosis and treatment.

To date, the treatment of depression has been on an individual basis. Numerous reasons exist, however, for the cessation of individual treatment regimes including all of those factors which ordinarily input to a "cost-benefit" analysis at an individual level (likelihood of further improvement, severity of illness, medication side-effects, etc.).

Thus, it appears that, in view of the overall burden depression creates for society - particularly the financial burden - alternative means of treating depression need to be explored. For example, evidence exists in support of the efficacy of maintenance chronic therapy. Under this theory, the clinical goal would be the maintenance of euthymia, not repetitive treatment of recurrent episodes which may contribute to a deteriorating lifetime course.

Under this theory, however, figures appear to indicate that it may only be viable to treat a portion of the depression-diagnosed population in this way, perhaps, with targeted interventions at subgroups at risk of adverse outcomes (in particular, recurrence). There is, therefore, a need to be able to accurately and effectively identify subgroups of the depression population at high risk of adverse health outcomes.

SUMMARY OF THE INVENTION

The present invention involves a computer-implemented method for generating a model to identify at risk patients diagnosed with depression, information about patients existing in a claims database, said method comprising the steps of 1) processing, based on predetermined criteria, the patient information in the claims database to find and extract claims information for a group of depression patients; 2) defining, using the information available in the claims database, events relevant to depression; 3) processing the extracted claims information and the defined events to create files containing event level information; 4) defining a time window for providing a timeframe from which to judge whether events should be considered in subsequent processing; 5) defining a set of variables as potential predictors; 6) processing the event level information, using the time window and the set of variables, to generate an analysis file; and 7) performing statistical analysis on the analysis file to generate a prediction model, said prediction model being a function of a subset of the set of variables.

Another aspect of the present invention involves a computer-implemented method for identifying, using the generated model, at risk patients diagnosed with depression, said method comprising the additional step of applying the prediction model to a processed claims database to identify and output a file listing the likelihood of each patient having an adverse health outcome.

BRIEF DESCRIPTION OF THE DRAWINGS

The invention is best understood from the following detailed description when read in connection with the accompanying drawing, in which:

Figure 1A is a high-level flowchart illustrating an exemplary overall process of the present invention.
Figure 1B is a high-level flowchart illustrating an exemplary process of the application of the present invention.

Figure 2 is a high-level block diagram illustrating three exemplary sources of information suitable for use with the present invention.

Figure 3 is a data structure diagram which shows an exemplary format in which the information from the sources of Figure 2 are stored in a research database.

Figure 4 is a data structure diagram which shows an exemplary format for an event level file generated during the process shown in Figure 1.

Figure 5 is a data structure diagram which shows an exemplary format for an analysis file generated, in part, from the event level file shown in Figure 4 and during the process shown in Figure 1.

Figure 6A is a time-line diagram which shows a first exemplary time window scheme suitable for use in processing the data from the event level files shown in Figure 4.

Figure 6B is a time-line diagram which shows a second exemplary time window scheme suitable for use in processing the data from the event level files shown in Figure 4.

Figure 7A is a table which shows experimental results using a hospitalization (HL) indicator with the Scheme 1 shown in Figure 6A.

Figure 7B is a table which shows experimental results using a High Cost indicator with the Scheme 1 shown in Figure 6A.

## DETAILED DESCRIPTION OF THE INVENTION

### Overview

The present invention is designed to identify, in a predetermined population of depression patients, those patients at high risk of adverse health outcomes. The identification of this high risk subgroup being an initial stage in attempts, e.g., targeted interventions, to prevent and/or improve their health outcome.

Initially, one or more sources of information are required which allows for the identification of an initial population of depression patients. Examples of sources include health care providers such as doctors, hospitals and pharmacies which all keep records for their patients. The individual records for each of these providers, however, may be scattered, difficult to access, and/or have many different formats.

On the other hand, a more comprehensive source containing this type of information exists in the health care claims records of any given benefits provider.

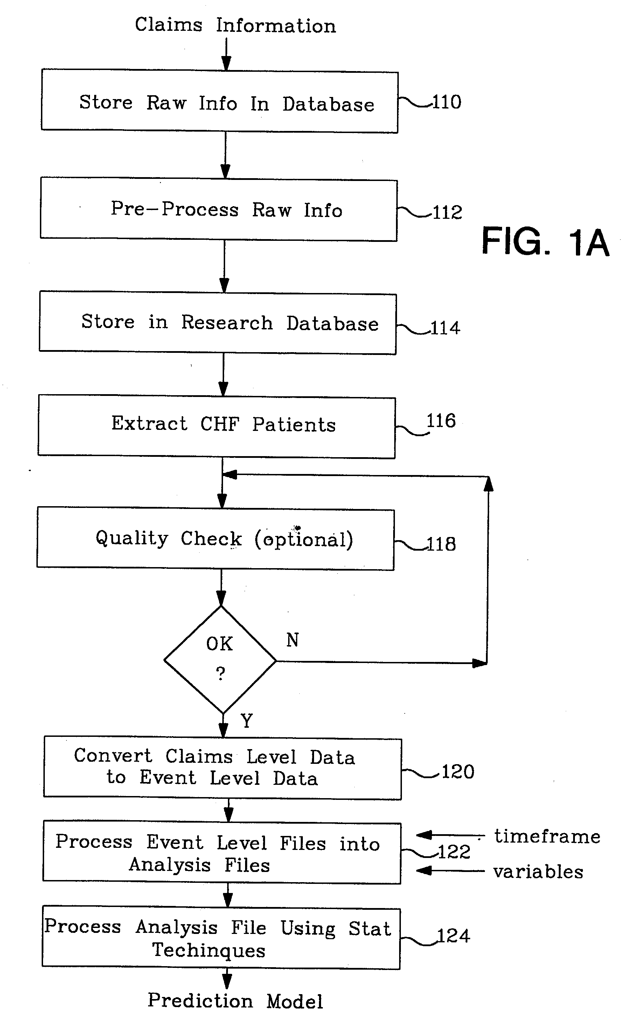

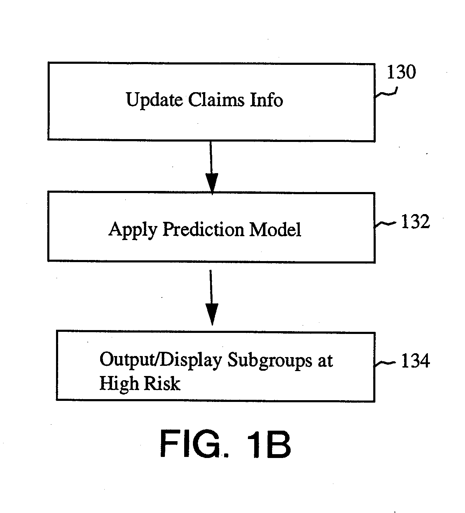

Turning to the figures, Figure 1A is a high-level flowchart illustrating an exemplary overall process of the present invention. As illustrated in Figure 1, the "raw" claims information is received and stored in a database (e.g., DB2 format) represented by block 110. In the world of claims processing, before this database of "raw" information can be useful, some pre-processing, step 112, is generally performed which may include rejecting claims, reconciling multiple claims and so on. The output of this preprocessing step, represented by block 114, is a "cleaner" database now stored, in the exemplary embodiment, in SAS format.

SAS is a well known format and software package produced by SAS Institute, Inc. of Cary, North Carolina. It should be noted that other data processing and storage formats, as appreciated by those skilled in the art, could be used in the storage and processing of data.

It should also be noted that SAS formats, programming techniques and functions are more fully described in the SAS/STAT User's Guide, Version 6, Fourth Edition, Volumes 1 and 2, 1990 and the SAS Language: Reference, Version 6, First Edition, 1990 which are both herein incorporated by reference for their teachings regarding the SAS language, SAS programming, functions and formats.

Moreover, the SAS routines used for processing information as part of the present invention are used for computational operations, executed on a computer and stored on a storage medium such as magnetic tape, disk, CD ROM or other suitable medium for purposes of storage and/or transportability. The stored software can then be used for running a computer.

The claims records of the benefits provider, although containing important information, may not be organized in a manner for efficient analysis. Thus, the next step is to perform another processing step (e.g., screening for depression patients, age, etc.), represented by block 116, to transform the "raw" data into a more appropriate and useful database. That is, the output data from the processing (i.e., extraction) step is a subset of the "raw" information and represents an initial universe of depression patients upon which further processing is performed.

A next step, which is optional, is to perform a "quality check" on the initial universe of depression patients. This step is somewhat subjective. This processing step, represented by block 118, using intermediate output files, performs a refinement of the extracted information by, for example, checking to see if an imbalance exists in the extracted information such as all claims are from individuals over 60 years of age or all claims are from men. This step, essentially a common sense check, can be performed as many times as necessary to ensure the integrity of the database data. At this point, the database data exists at the claim level.

The information existing at the claim level provides various information in the form of raw data elements. From the claims level data, the next processing step, represented by block 120, creates new files (e.g., primary file 1 and primary file 2) by reformatting the information into an event level format.

Before this occurs, a set of events (e.g., doctor visit for depression) relevant to depression are defined using a combination of both the raw data elements available from the claims information and clinical knowledge about depression. With these events defined, the claims level information is used to created new files based on events rather than claims. Having the information in an event level format is an important aspect of the present invention in that, among other things, it allows for added flexibility in subsequent analysis.

As depicted by block 122, further processing is performed on the event level data to generate an analysis file. In particular, the processing is performed using input information representative of a sliding time window and a plurality of variables. The time window input limits the time periods in which the events from the primary files are considered. That is to say, the time window is used to identify an analysis region and a prediction region where activity in the analysis region is used to predict some predetermined outcome in the prediction region. The selection of variables, both dependent and independent, for analysis, is an important step impacting the accuracy of the final prediction model. The dependent variables are representative of the desired result (i.e., an adverse health outcome to be predicted); whereas, the independent variables are representative of predictors. This processing step, step 122, can be easily reprogrammed, via the input parameters, for various time window adjustments as well as various variable modifications. The analysis file generated at this step is a member level file which means it is broken down by member.

With the analysis file in hand, a model or technique for identifying high risk subgroups is determined. That is, as represented by step 124, the analysis file is used to develope an identification technique represented by an equation incorporating a subset of the initial variables programmed into the above-mentioned processing step. The resulting subset are those variables which best reflect a correlation to adverse health outcomes, consequently, resulting in substantial use of health care resources (e.g., funds). It should be noted that the determination of the initial as well as the final variables is an important aspect of present invention as the variables may significantly impact the accuracy of the identification of the subgroup.

The above model for identification can be developed, step 124, in various ways using statistical techniques. The technique used in the exemplary embodiment of the present invention for generating the model is multiple logistic regression.

Figure 1B is a high-level flowchart illustrating an exemplary process of the application of the present invention. Having developed the model, as shown in Figure 1A, it can then be applied to updated claims data, step 132, or to other databases of depression patients (e.g., claims information for other benefits providers), in order to identify at risk patients diagnosed with depression, step 134, allowing for various types of targeted intervention to maximize the effective allocation of health care resources.

Exemplary Embodiment of the Invention

Although the present invention is illustrated and described below with respect to specific examples of a method and system for identifying depression patients at high risk for adverse health outcomes, the invention is not intended to be limited to the details shown. Rather, various modifications may be made in the details within the scope and range of equivalents of the claims and without departing from the spirit of the invention.

As mentioned, the present invention is designed to identify patients with depression at high risk of adverse health outcomes. The identification of this high risk subgroup being the first step in being able to try different treatment techniques (e.g., targeted interventions).

Initially, a source of information is required which allows for the identification of a population of depression patients. A comprehensive source containing this type of information exists in the health care claims records of many benefit providers. As is known, claims for drugs, doctors and hospitals are received and processed for payment/reimbursement. In the exemplary embodiment of the present invention, this claims information is entered into a DB2 database on a benefits provider's computer system (not shown).

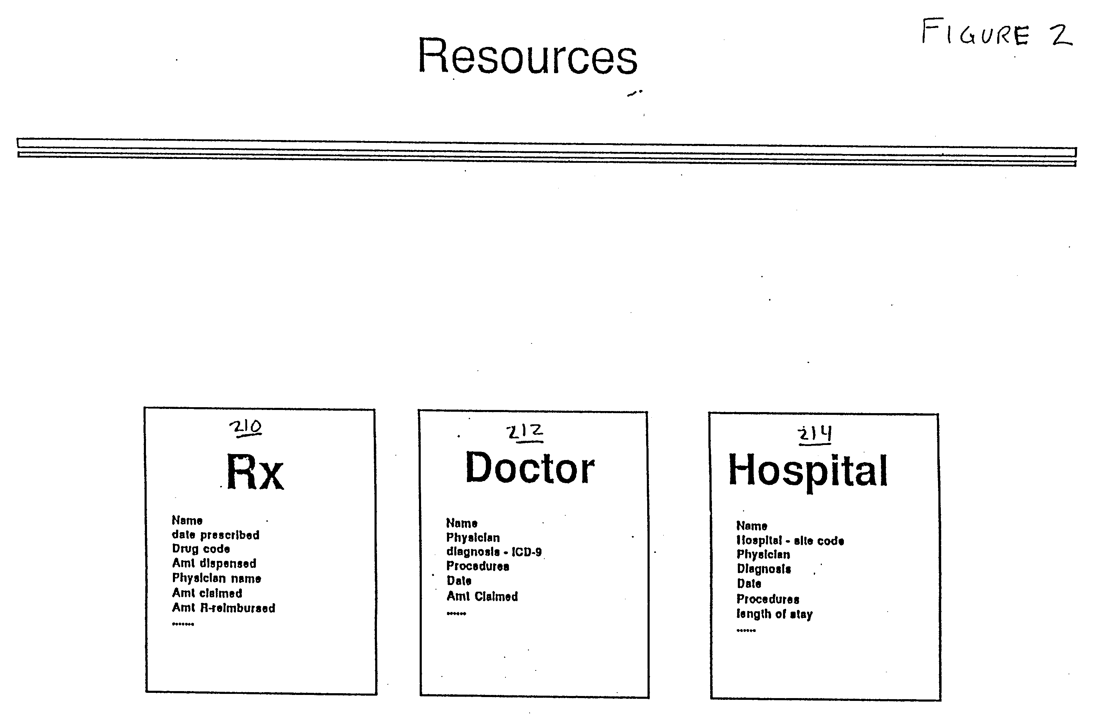

Figure 2 is a high-level block diagram illustrating three exemplary sources of information suitable for use with the present invention. As illustrated in Figure 2, the claims information of such a provider would typically include three sources: pharmacy claims (Rx) 210, doctor (DR) claims 212, and hospital (HL) claims 214. As listed on the blocks representing the claims information, many types of information would be available from the respective claims including drug codes, physician's names, diagnosis codes, procedures, various dates and other important information. Much of this information is referenced using codes, such as drug codes, procedure codes and illness codes. Appendices I-VI provide listings of various codes used with the present invention. These codes were selected for processing purpose of the present invention from a voluminous source of codes and, as will be appreciated by those skilled in the art, may be modified to include/exclude codes deemed more/less useful at the various stages of processing.

The DB2 database represents a source of "raw" data elements which require processing. A first step in processing this raw data is to perform data integrity checks (e.g., rejected or reconciled claims). Subsequently, the data is routinely download into a "research" database. The research database is a claims level database in SAS format.

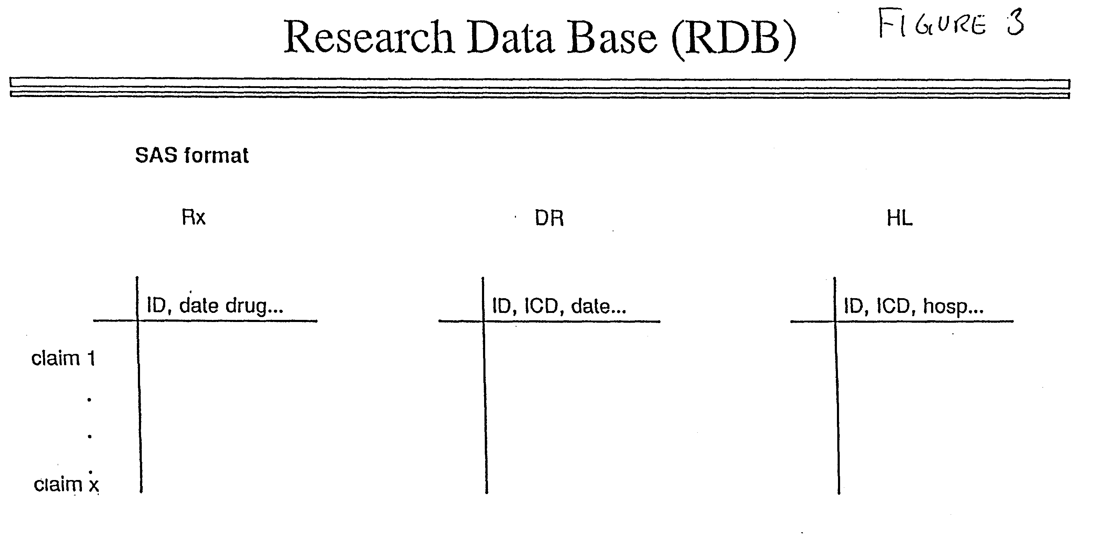

Exemplary formats, for each of the Rx, DR and HL claims, of the records contained in the research Database, are shown in Figure 3. As shown in Figure 3, claims are listed from claim 1 to claim x and the appropriate information, for the particular service provider (e.g., Rx) being claimed, is also presented.

Once in SAS format, SAS procedures process the information to 1) extract patients with depression (step 116), 2) process the claims level information into event level information (step 120), 3) using predetermined variables and timeframe schemes, generate analysis files for analysis purposes (step 122) and 4) create a prediction model as a function of those variables most reflective of the correlation to an adverse health outcome (step 124).

It should be mentioned that, from a statistical perspective, an important consideration in developing prediction models from datasets is sample size. To maximize the integrity of the prediction model, sample size is an important factor. Prevalence of depression is reported to be approximately 5%, however, sample sizes required to determine prediction equations depend on the magnitude of association between variables. As these associations are unknown, all patients within any individual plan are initially included.

The first step, extracting patients with depression (step 116), uses various parameters to define which patients qualify for the overall initial universe of depression patients to be considered.

For example, in the exemplary embodiment of the present invention, only patients having a continuous enrollment with the benefits provider of 12 months or longer and having a claim for depression or treatment with anti-depressant medication are eligible. Of course, these criteria are exemplary and could be modified such that 24 months or 6 months of enrollment is satisfactory or that an individual must be 18 years of age. In the exemplary embodiment of the present invention, the claims extraction step, step 116, extracts all claims data for patients with either an appropriate code for depression (see Appendix I) or for treatment with an antidepressant drug (see Appendix III).

It should be noted that in the health care industry various codes are used in claims information for indicating which procedures, treatments, diagnoses, drugs, etc. are being claimed. For the exemplary embodiment of the present invention, the selected codes are shown in Appendices I-VI. These codes were found in Physician's Current Procedural Terminology (CPT), American Medical Association (1995) and St. Anthony's ICD-9-CM Code Book (1994) which are both herein incorporated by reference for their teaching of codes and sources of codes. As will be appreciated by those skilled in the art, any set of codes, representative of the various procedures, treatments, diagnosis, drugs, etc. relevant for use with the present invention would suffice. Reference to such codes occurs throughout this specification.

Subsequent to the claim extraction step, the claim adjustment and integrity checks are optionally performed, step 118. To do so, from the dataset defined above, intermediate output files are generated which contain sets of frequency counts for processing purposes. In the exemplary embodiment of the present invention, intermediate output files for the following characteristics are generated for review:

a. frequency counts of unique members by sex, age groups (0-9, 10-19...) and enrollment duration by months including:

i) Tables showing count of members by sex, ii) Table showing count of members within age groups, iii) Table of counts of age groups broken down by sex, iv) Table of enrollment duration by months i.e., 1 month to maximum number of months possible.

b. frequency counts of ICD codes for depression (Appendix I),.i.e., number of members having at least one hit with each of the ICD codes in Appendix I any level ii) as first code.

c. frequency counts of anti-depressant drugs (Appendix II):

i) number of members who have at least one claim for each of the drugs in Appendix III.

d. count of members who became eligible for processing due to ICD code only, by drug only, and by both ICD code and drug.

e. frequency counts of numbers of all claims within each file (HL, DR, Rx) by member.

f. frequency counts of ICD codes (use only the first 3 digits of ICD codes) of any nature in DR (any position) and HL files - at least the top 10 with frequency of each. i.e., 2 tables one each for DR and HL files.

g. frequency counts of hospitalizations by calendar month. Counting calendar month backward from last month of eligibility or data availability. The last month for which data is available will be month 1, the penultimate month with be month 2 etc.

h. frequency counts of procedures related to depression (CPT codes, Appendix II).

i. frequency counts of all CPT codes (to the level of the first 3 code digits) - at least the top 10.

The above frequency counts for use in performing preliminary evaluations as to the integrity of the data is exemplary and could be modified to include/exclude parameters which are shown to be more/less useful.

With this information, a "quality check" is performed on the initial universe of depression patients to make sure that the final results, i.e., prediction model, is not unreasonably skewed due to bogus input information. This processing step, block 118, using intermediate output files, allows for a refinement of the extracted information by, for example, checking to see if an imbalance exists in the extracted information such as all claims are from individuals over 60 years of age, all claims are from men, or other data imbalances which would otherwise taint the integrity of a prediction model. Step 118, in the exemplary embodiment, is performed manually by viewing the intermediate output files. It is contemplated, however, that using various threshold values, the frequency counts can be automatically scanned for a potential imbalance.

Having now extracted and refined the claims level information according to various predetermined criteria deemed relevant for subsequent processing purposes, the information is converted into an event level format.

To provide processing flexibility, particularly in assigning time windows for analysis, the above-mentioned second step (i.e., converting the claims level information into event level information, step 122) is employed to generate two primary data files from which an analysis file can be created.

In the exemplary embodiment of the present invention, primary data file 1 is a member level file and contains all data of a static nature (i.e., not time sensitive) such as 1) Member Key, 2) Date of birth, 3) Gender, 4) First available date of enrollment (i.e., start of dataset (1/1/92) or enrollment date), 5) End data of enrollment (i.e., end of dataset or last date of enrollment), 6) Date of first depression event (first prescription for antidepressant or depression hospitalization), 7) Date of last hospitalization, 8) Number of records in events file (primary file 2), and 9) Mode of entry into the dataset (e.g., i) Anti-depressant drug only, ii) Depression diagnosis only, iii) Both anti-depressant drug and depression diagnosis).

Primary data file 2 is an events level file with a record for each event ordered by member and the chronological date of the event, in the present invention, presented in descending order of event date.

It should be noted that an event, sometimes referred to as an episode, is an occurrence which, based on clinical knowledge, is deemed relevant to depression. Having knowledge of what raw data elements are available from the claims, a set of events is defined directly or indirectly from the data elements where events can be based on an individual data element, combination of data elements or derived from individual or multiple data elements.

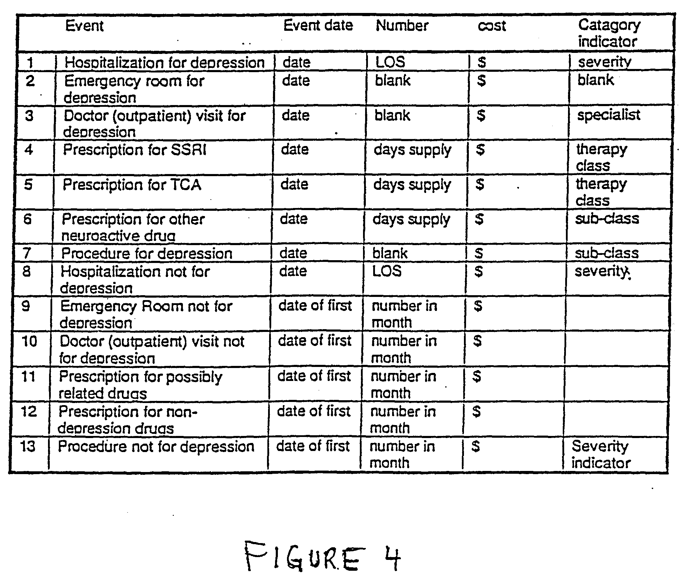

Figure 4 is an exemplary list of events and format for primary file 2 (an event level file). As shown in Figure 4, the entries provided include:

1. Hospitalization for depression

    a. Any hospital claim identified by hospital site code.
    b. Having a from and through duration of at least 1 day.
    c. Having ICD 9 code.
    d. Depression ICD 9 code occurring at any position.
    e. Illness indicator (Appendix V) 1 = major illness, 2 = suicide, 3 = major illness and suicide. 0 = everything else.

2. Emergency room for depression

    a. Emergency room visit identified by emergency room site code.
    b. Having ICD 9 code (see Appendix I).

3. Doctor (non-hospital) visit for depression

    a. Any doctor claim.
    b. Having ICD 9 code (see Appendix I).
    c. Category : Psychiatrist =- 1, all others = 0.

4. Prescription for SSRI

    a. SSRI (selective serotonin re-uptake inhibitors) therapeutic class 5.51.3.
    b. Cost = 0 if generated from a hospital admission.
    c. Category indicator = blank

5. Prescription for (Tricyclic antidepressants) TCA or (Monoamine Oxidase Inhibitors ) MAOI

    a. Therapeutic classes 5.5.1.1 (tertiary amines), 5.5.1.2 (secondary amines), 5.5.1.4 (Monoamine Oxidase inhibitors). AND 5.5.2
    b. Cost = 0 if generated by a hospital admission
    c. Category indicator = therapeutic class 1 = 5.5.1.1, 2 = 5.5.1.2, 3 =5.5.1.4, 4 = 5.5.2

6. Prescription for other neuroactive drug (From Rx file)
7. Procedure for depression (from DR or HL files) Category:
    CPT codes or ICD procedure

| | |
|---|---|
| 0 = Psychotherapy | All CPT and ICD codes in Appendix II not listed below. |
| 1 = Diagnostic | 90801, 90820, 90825, 90830, 90862 |
| | 94.0x, 94.1x, 94.21, 99.22, 94.23 |
| 2 = Shock therapy | 890870, 908712 |
| | 94.24, 94.26, 94.27 |

For this entry, costs are assigned to the doctor visit or hospitalization in which the procedure occurred.
8. Hospitalization not for depression
It should be noted that items under entry 8 could have been performed for a condition other than depression although these patients got into the cohort by virtue of receiving a depression diagnosis or receiving and antidepressant at some time making it likely these procedures were for depression.

    a. All hospitalization having from and through dates of at least one day duration.
    b. Major illness ICD 9 codes (see Appendix V).
    c. Category as in 1 above (1 = major, 2 = suicide, 3 = both, 0 = all others)

Counts for entries 9-13 are aggregated for each month. The date is that for the first occurrence of the identified events. In the number field, the number of identified events occurring in that month are summed.
9. Emergency room not for depression
    a. Emergency room visit identified by Emergency room
10. Doctor (outpatient) visit not for depression

    a. Any doctor visit.
    b. Excluding visit with a depression diagnosis (Appendix I) i.e., not in 3/above.

11. Prescription for possibly related drugs
    Drugs identified in Appendix IV
12. Prescription for all other (non-depression) drugs
    All drugs not included in Appendices III or IV.
13. Procedure not for depression (from Dr and HL files)
    a. Category indicator 1 = major procedures, 2 = minor procedure (see Appendix IV).

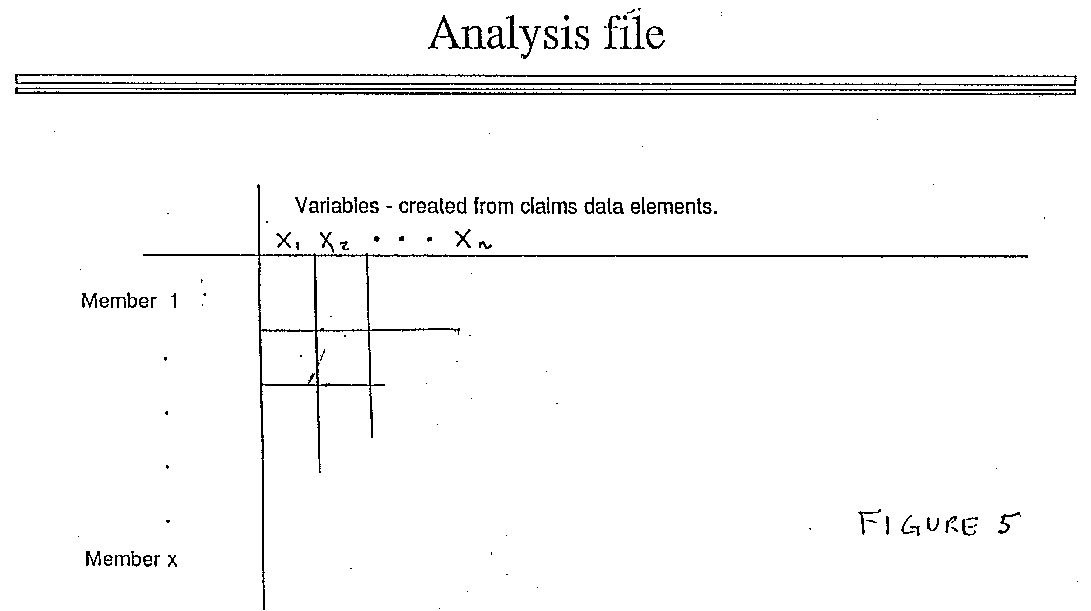

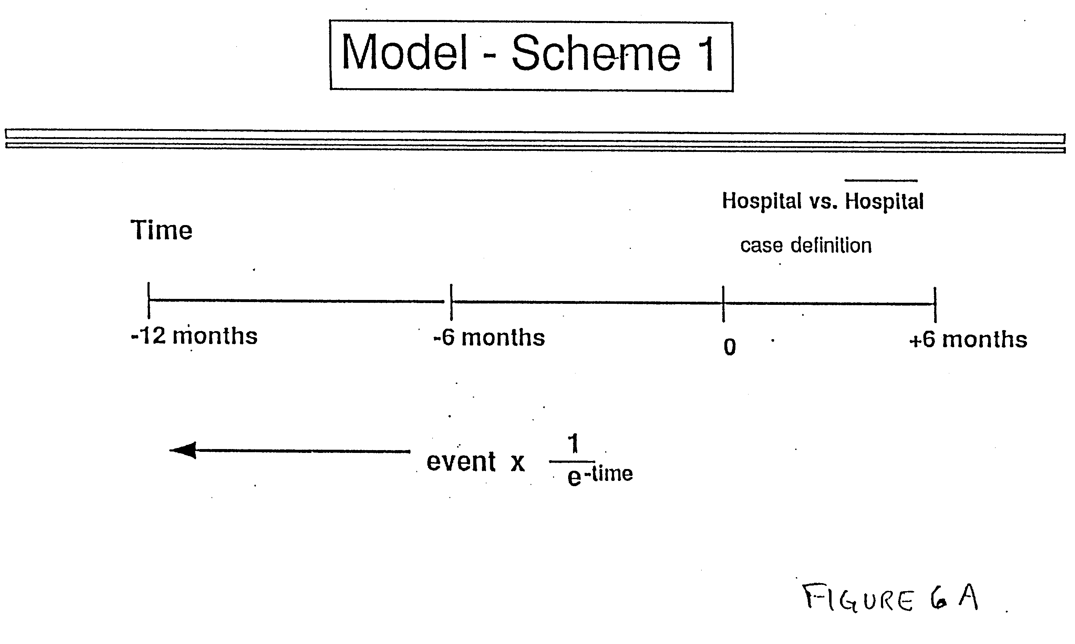

After generating the two primary files using the above described instructions, corresponding to step 120 of Figure 1, further processing is performed on the event level data to generate an analysis file, step 122. An exemplary format for the analysis file is shown in Figure 5. As shown, the format of the analysis file includes a list of members in a first column of a table. Across the top of the table is a list of variables, described in detail below. And, the body of the table provides indications as to a member's relation to a listed variable.

In particular, the processing from the primary files to the analysis files includes an algorithm defined, in part, by a time window and a plurality of variables. The algorithm can be re-programmed for various time window adjustments as well as variable modifications. The analysis file generated at this step is a member level file (i.e., organized with respect to members). The main analysis files are member level files derived from the information in the primary files.

Each main analysis file is created to take into account a single reference time window of censored events and prediction window of interest for that file. Each new time window applied to the data, in the exemplary embodiment, requires another main analysis file.

To generate the analysis file, a time window scheme, along with a plurality of variables, is applied to the event level data.

Discussing the variables first, included in the processing are both independent and dependent variables. The indepedent variables basically represent potential predictors of the adverse health outcomes; whereas, the dependent variables basically represent the adverse health outcome to be predicted.

To determine exemplary independent variables for step 122, as many of the original data elements as possible are used, assuming nothing about depression. Then, based on clinical knowledge, additional variables are created. Furthermore, combinations of the data elements and/or variables, based on clinical knowledge, are used as variables.

Finally, some variables may be created and used based on their potential utility as a leverage point in disease management.

It should be noted that, for purposes of a cost heirachy, the following rules were used in the exemplary embodiment of the present invention.

1. Only hospitalizations for depression can spawn other events.
2. Hospital costs include all Rx, procedure, physician charges.
3. Hospital visits can generate Rx and procedure events with costs set to zero (included in hospital cost).
4. Hospital visits cannot generate separate doctor visit events.

In the exemplary embodiment of the present invention, the plurality of variables currently used by step 122 in the SAS routine for generating an analysis file from an event level file are shown below in Table 1. In Table I, although the abbreviations should be self-evident, by way of example, some abbreviations are as follows: "DEP" means depression, "HL" means hospitalization, "#" means number, "MOS" means months, "OTH" means other, "ER" means emergency room, "RX" means prescription, "SUP" means supply, "PROCS" means procedure, and "TOT" means total.

## Table 1

1. "DEPENDENT CODE"
2. "DEPRESSION HL INDICATOR"
3. "# OF MOS AVAILABLE FOR ANALYSIS"
4. "AGE AT TIME OF CUTOFF"
5. "FEMALE INDICATOR"
6. "TOTAL COST DURING ANALYSIS PERIOD"
7. "# OF DEPRESSION DRUG CLASS SWITCHES"
8. "DEPRESSION DRUGS DAYS SUPPLY"
9. "# DEP HLS"
10. "# DEP OTH HLS"
11. "# DEP HLS AND MAJOR ILLNESS"
12. "# DEP HLS AND SUICIDE"
13. "# DEP HLS AND MAJOR ILLNESS AND SUICIDE"
14. "# DEP HLS AND DEP RELATED CODE"
15. "# DEP HL LENGTH OF STAY"
16. "# DEP ER VISITS"
17. "# DEP DR VISITS"
18. "# DR/PSYCHIATRIST VISITS"
19. "# RX FOR SSRI"

20."# DAYS SUP OF SSRI"

21."# RX FOR TCA"

22."# RX TCA: TERTIARY"

23."# RX TCA: SECONDARY"

24."# RX TCA: MONO OXI INHIBITORS"

25."# RX TCA: ALL OTHER TYPE"

26."# DAYS SUP OF TCA"

27."# DAYS SUP OF NEUROACTIVE (NA)"

28."# DYS SUP OF NA: ANXIOLYTICS AND SEDATIVE"

29."# DAYS SUP OF NEUROACTIVE: ALL OTHER"

30."# DEPRESSION PROCS"

31."# DEP PSYCHOTHERAPY PROCS"

32."# DEP DIAG PROCS"

33."# DEP SHOCK THERAPY PROCS"

34."# OTH HOSPITALIZATIONS"

35."# OTH ALL OTH HLS "

36."# OTH HLS AND MAJOR ILLNESS"

37."# OTH HLS AND SUICIDE"

38."# OTH HLS AND MAJOR ILLNESS AND SUICIDE"

39."# OTH HLS AND DEP RELATED CODE"

40."# OTH HL LENGTH OF STAY"

41."# OTH ERS"

42."# OTH DR VISITS"

43."# RX FOR RELATED DRUGS"

44."# DAYS SUP RX FOR RELATED DRUGS"

45."# RX FOR ALL OTHER"

46."# DAYS SUP RX FOR ALL OTHER"

47."# PROCS NOT FOR DEP"

48."# PROCS FOR MAJOR ILLNESS"

49."# PROCS FOR MINOR ILLNESS"

50."% DEP HL COST OF TOT COST"

51."% DEP ER COST OF TOT COST"

52."% SSRI COST OF TOT COST"

53."% TCA COST OF TOT COST"

54."% NEUROACT COST OF TOT COST"

55."% OTH HL COST OF TOT COST

56."% OTH ER COST OF TOT COST"

57. "% OTH DR COST OF TOT COST"

58. "% OTH RELATED RX COST OF TOT COST"

59. "% OTH ALL OTHER RX COST OF TOT COST"

60. "# COST OF DEP RELATED EVENTS"

61. "# COST OF OTH RELATED EVENTS"

62. "# COST DEP DRUGS FROM ALL DRUG COSTS"

63. "# COST OTH DRUGS FROM ALL DRUG COSTS"

64. "# COST DEP DRUGS FROM ALL COSTS"

65. "# COST OTH DRUGS FROM ALL COSTS"

66. "# SSRI COST FROM DEP DRUGS COSTS"

67. "% TCA COST FROM DEP DRUGS COSTS"

68. "% NEUROACTIVE COST FROM DEP DRUGS COSTS"

69. "DEP HL IN LAST 12 MOS INDICATOR"

70. "DEP ER IN LAST 12 MOS INDICATOR"

71. "MOS BETWEEN 1ST AND LAST EVENT"

72. "MOS SINCE FIRST DEP EVENT"

73. "MOS SINCE LAST FIRST DEP EVENT"

74. "MOS OF DATA USED FOR ANALYSIS"

75. "DEP RX COMPLIANCE MEASURE"

76. "# DEP HL BY GENDER INTERACTION"

77. "# DEP ER BY GENDER INTERACTION"

78. "# DEP DR VISITS BY GENDER INTERACTION"

79. "# RX SSRI BY GENDER INFORMATION"

80. "# RX TCA BY GENDER INTERACTION

81. "# RX NEUROACTIVE BY GENDER INTERACTION"

82. "# DEP PROCS BY GENDER INTERACTION"

83. "# OF UNIQUE GENERALIST DRS USED"

84. "# OF UNIQUE PSYCHIATRISTS USED"

Turning to the dependent variables, potential dependent variables, for example, contemplated for use with the present invention as results to be predicted include:

1. Hospital (HL) admission or emergency room (ER) visit for depression. This is a dichotomous variable which is referred to as the HL (or ER) indicator such that HL (or ER) = 1 if an admission or ER visit occurred, otherwise the indicator equals 0.

2. Highest 10% of resource utilization measured in dollars. Resources counted from time of cost in the top 10% of the first depression diagnosis or receipt of first antidepressant (in the record) + 1, 3 and 6 months - separate analyses for each time period. Again, this is a dichotomous variable referred to as the High Cost indicator such that if patient in top 10%, Host Cost = 1, otherwise High Cost = 0. The High Cost indicator, in the exemplary embodiment, could also be defined as the distribution of total cost per member (PMPM) in the prediction region (B to C) is used to define this variable. The High Cost indicator is set to 1 for the 10% of members with the highest PMPM in the Total Cost distribution and set to 0 for all others.

3. Any hospital admission for attempted suicide - identified by claim related to any of the ICD 9 codes 300.9 or 800-999. As those of ordinary skill in the art will appreciate, using attempted suicide as a dependent variable may only provide useful results if there exists a sufficient number of occurences to do so.

Although only three dependent variables are listed above, as those of ordinary skill in the art will appreciate, other known or yet unknown variables may also suitably serve as a dependent variable within the scope of the present invention.

Turning to the time window aspect of the generation of the analysis file, it should be noted that there is one analysis record for each selected member.

In the present invention, several schemes, as described below, have been developed for defining prediction zones and censoring data to create the analysis file. That is, a time window basically defines a prediction zone or region and an analysis region where the analysis region in where activity is used to predict something in the prediction zone. Additional time window schemes may also adequately serve the present invention.

For purposes of explanation, the time that the claims history covers is referred to as the time window that starts at point 'A' and ends at point 'C'. The time interval is divided into analysis and prediction regions by point 'B' such that A<B<C.

By way of example, Jane Doe's analysis record is based on claims from 1/1/91 through 6/30/93. Therefore, A=1/1/91, C=6/30/93 and B can be selected somewhere in between, such as 12/31/92. Generally, A is defined based on the data extraction protocol (i.e., from when the data is available) and C is defined by the last day for which the member is still enrolled and eligible for the benefits. Of course, variations of those general points of definition could be selected within the scope of the present invention.

The definition of B is important. In the present invention, two basic definitions of B were devised in order to maximize the accuracy of the prediction model. Although, as would be understood by those skilled in the art, alternative definitions of B are contemplated.

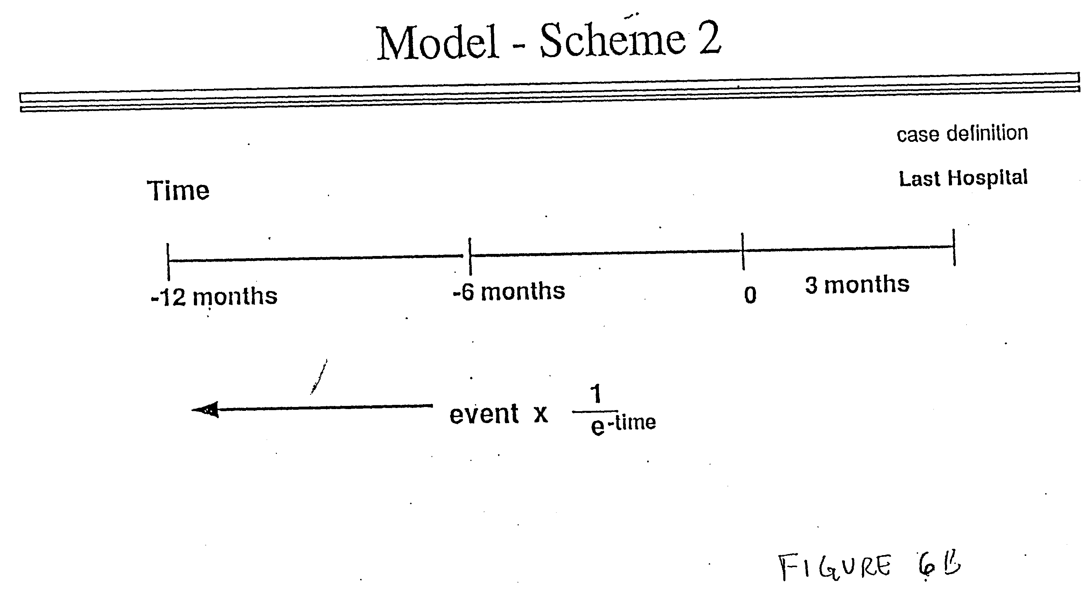

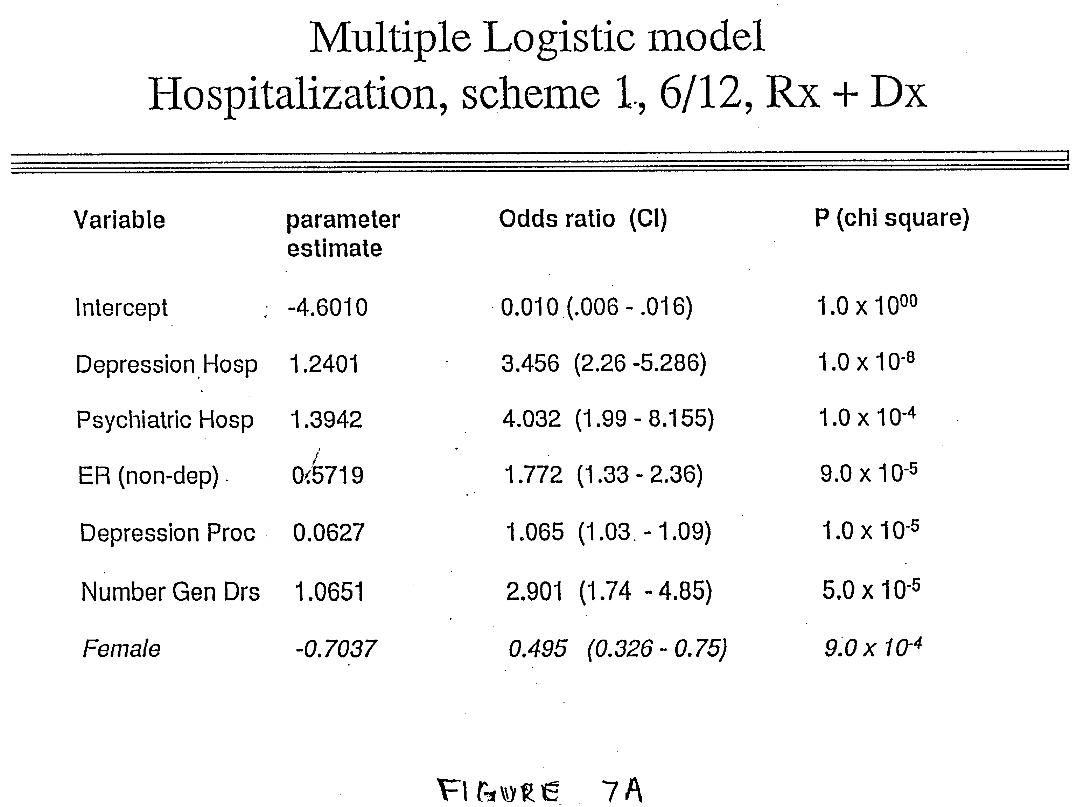

Figure 6A is a first exemplary time window scheme, referred to as Scheme 1, for use in processing the data from the event level files shown in Figure 4.

In Scheme 1, the event prediction region is set from B to C such that B=C-(x# of months) for all the members in the analysis. For example, if a 6-month depression hospitalization (HL) model (i.e., HL is used as a dependent variable) is to be built then B=C-(6 months). In Jane Doe's example, B would equal 12/31/92. Therefore, only data covering from A through B (1/1/91-12/31/92) is used to predict the depression HL in the 'next 6 months'. The phrase 'next 6 months' in this context implies that the time point B is "NOW" and any time after it is in the FUTURE and any time before it is in the PAST. This is a key concept of Scheme 1 and is important to understanding the prediction model implementation and application.

As additional explanation, when a variable is defined such as '# of Psychotherapy Visits in the LAST 6 Months', that means that the count for this variable is based on claims from [(B-6 months) to B] for every member in the analysis. It should be noted, however, that point B may vary with every member in the analysis population.

An alternative to Scheme 1, and referred to as Scheme 2, is illustrated in Figure 6B which shows a second exemplary time window scheme for use in processing the data from the event level files shown in Figure 4.

A difference between Scheme 1 and Scheme 2 is the definition of the prediction region for members which have at least one depression hospitalization or emergency room visit (HL/ER). The prediction region starting at point B, in Scheme 2, is defined in multiple passes over each member's record. Turning again to Jane Doe's analysis record (from 1/1/91 through 6/30/93, A=1/1/91, C=6/30/93) to illustrate how this aspect works for defining point B, assume that Jane Doe was hospitalized for depression three times: on 4/1/91, 4/1/92, and 4/1/93.

Point B is set equal to the date of the first depression HL/ER - 1 month or set equal to point C if a member never had depression HL/ER in their claims history. For Jane Doe, B=4/1/91. In the exemplary embodiment of the present invention, moving back one month from the HL date is performed to simulate the model application environment. There would probably be at least 30-day lag from model scoring to the disease management actions based on the scoring reports. Thus, in Jane Doe's record B=4/1/91-(1 month)=2/28/91. Jane's record, in this case, would not be used in the model building because the time span of the analysis region is only two months--less than the exemplary six month data history requirement.

Repeating steps 1 and 2 using second (or third or...) HL date to set point B, Jane Doe's record would eventually make it into model building on the second and third pass. This process, in the exemplary embodiment, terminates after three or four passes since there would probably be very few members with five or more depression HL/ERs in the study population.

It should be noted that the consequence of repeated modeling introduces added complexity of setting up additional independent variables. An important advantage, however, of Scheme 2 is that the prediction HL/ER rate would likely be higher than in Scheme 1.

In still another alternative embodiment, analysis weights which reflect proximity to the event to be predicted can be used, for example, within 3 months x 1, 3-6 months x .75, 6-9 months x. .5, 9-12 months x .25, greater than 12 months x .125. Other suitable weighting techniques, as will be appreciated by those skilled in the art, could be used. These type of weighting techniques may be used with either Scheme 1 or Scheme 2.

Therefore, given a selected time window scheme and an appropriate set of predetermined variables, the processing step of 122 generates the analysis file.

Using the analysis file, the model for identification/prediction can then be developed in various ways using statistical techniques. In particular, the analysis file, now at a member level, is processed using statistical functions available in SAS. In the exemplary embodiment of the present invention, the statistical processing performed to generate the prediction model is multiple logistic regression. As will be appreciated by those skilled in the art, other statistical techniques may also be suitable for use with the present invention.

In the exemplary embodiment, the statistical processing, when applied to the analysis file, identifies variables which meet predetermined levels of significance (e.g., prpobability value < 0.05). These variables then form a prediction model which is a mathematical equation of the following form:

$$\text{Logit}(p) = a + bx1 + cx2...+ zxi$$

where x1...xi are the identified variables and a...z are there parameter estimates. An individuals probability (p) for the outcome under consideration is then determined using the following formula:

$$p = \text{e-logit}(p) / (1+\text{e-logit}(p)).$$

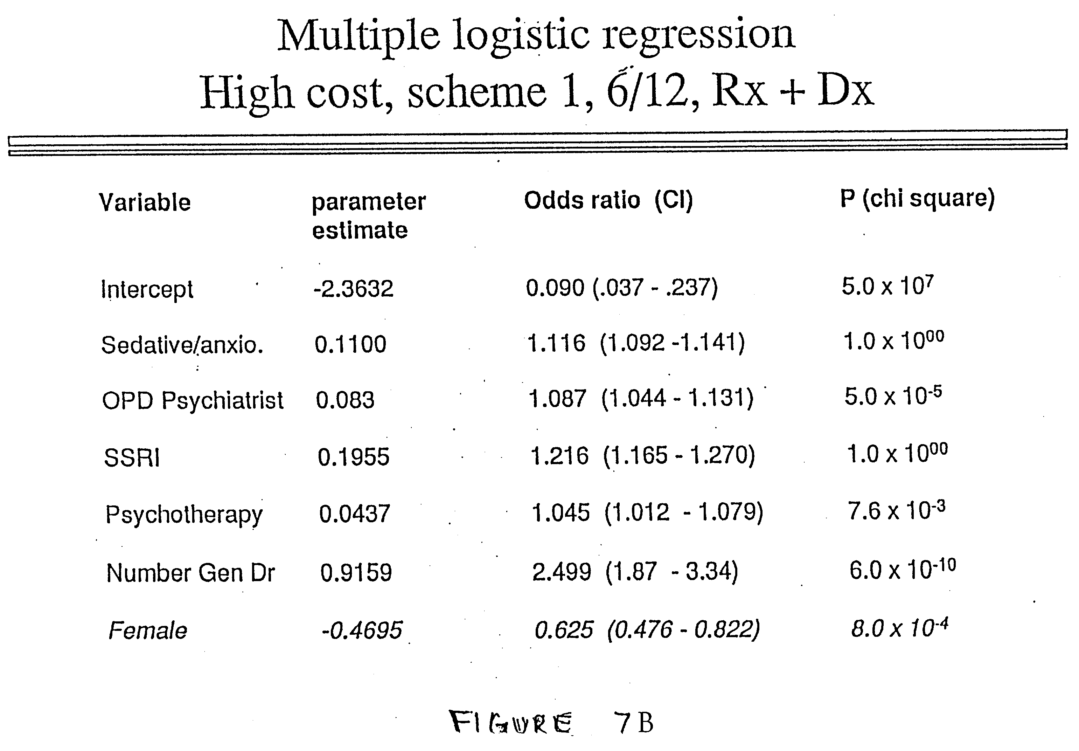

Figure 7A shows experimental results for a model based on Scheme 1 and using the HL indicator as a dependent variable. The resulting independent variables selected for the prediciton model include "FEMALE INDICATOR", "# DEP HLS", "# DEP ERS", "# DR/PSYCHIATRIST VISITS", "# DEP PROCS", and "# OTH HLS AND DEP RELATED CODE".

Figure 7B shows experimental results, including the dependent variables, for a model also based on Scheme 1 but using, as the dependent variable the High Cost indicator.

It should be noted that, although both experimental results indicate that six independent variables were used for the prediction model, more or less independent variables could be used based on their individual ability to accurately predict the selected dependent variable.

Next, the determined model is applied to the data. That is, because the prediction zone in the above processing was actually based on past data for analysis purposes, the model is now applied to the data such that a prediction zone is defined in the future. The determined model can be applied to the existing data, to the data as it is regularly updated or to other claims databases for other benefits providers. To do so, only the determined independent variables of interest need to be processed. Of course, as new claims databases are to be analyzed, the entire process can be repeated to generate a new model in order to determine if other variables may be better predictors. The output generated by applying the model is a file containing a list of all of the depression patients and a indicator representative of the likelihood that that patient will have an adverse health outcome (i.e., experience that defined by the dependent variable). This list can then be divided into subgroups such as in 5% or 10% increments of patients likely to have the adverse health outcome.

Applying the model to future claims data or other databases of depression patients or building a new model in a new database as described above, depression patients at high risk can be identified allowing for various types of intervention to maximize the effective allocation of health care resources for depression patients. Such intervention may take the form of 1) specific case management, 2) novel interventions based on subgroup characteristics, 3) high risk intervention, 4) high (relative) cost intervention, or 5) plan modification all adhering, of course, to the best practice guidelines.

Appendices I-VI follow.

## Appendix I

### Depression ICD-9-CM Codes

| ICD-9-CM Code | Description |
|---|---|
| 296.2x | major depressive disorder, single episode |
| 296.3x | major depressive disorder, recurrent episode |
| 296.5x | bipolar affective disorder, depressed |
| 296.82 | atypical depressive disorder |
| 298.0x | depressive type psychosis |
| 300.4x | neurotic depression (dysthymia) |
| 311.xx | depressive disorder, not elsewhere classified |

# Appendix II

## CPT-4 or ICD-9-CM Procedure Codes for Psychotherapy

### ICD-9-CM Procedure Codes:

| | |
|---|---|
| 9426 | Sub convulsive electroshock therapy |
| 9427 | Other electroshock therapy |
| 943-9439 | Individual psychotherapy |
| 44-9449 | Psychotherapy and counseling |

### CPT-4 Codes:

| | |
|---|---|
| 908xx | All psychiatric procedure codes |
| 90841-90844 | Individual medical psychotherapy |
| 90846-90849 | Family medical psychotherapy |
| 90855 | Interactive individual psychotherapy |
| 90857 | Interactive group psychotherapy |
| 90870-90871 | Electroconvulsive therapy |

## Apeendix III

## Antidepressants Agents
### (From the DPS national formulary, 1995)

Therapeutic class 5.5.1.1 (Tertiary Amines)
  amitriptyline
  doxepin
  imipramine
  trimipramine
  clomipramine

Therapeutic class 5.5.1.2 (Secondary Amines)
  desipramine
  nortriptyline
  amoxapine
  protriptyline

Therapeutic class 5.5.1.3 (Selective Serotonin Reuptake Inhibitors)
  paroxetine
  sertraline
  fluoxetine

Therapeutic class 5.5.1.4 (Other Antidepressants)
  amitriptyline/perphenazine
  trazodone
  burpropion
  venlafaxine

Therapeutic class 5.5.2 (Monoamine Oxidase Inhibitors)
  isocarboxazid
  phenelzine
  tranylcypromine

## Appendix IIIa
### Neuroactive drugs not for depression

all codes 5.x not in appendix above

Appendix IV

Drugs possible used in excess by patients with severe depression
DPS 1995 Formulary

Possible related drugs

51.     Analgesics and other medications for headache

9.1     Antacids
9.2     Antidiarrheal
9.3     Antispasmodic
9.4     Antiulcer
9.5     Laxiative
9.6     Other GI

11.1.1  Salicylates
11.1.2  Non-steroidal anti inflammatory drugs
11.3.1  Direct muscle relaxants
11.3.2  CNS muscle relaxant drugs
11.4    Other muscle relaxants

12.1.2  Multivitamins, fluorides, B2, Folic Acid, therapeutic vitamins
13.1.1  Prenatal vitamins
13.7    Oral contraceptives

15.2.1  Antihistamines
15.2.2  Decongestants
15.2.3  Combination antihistamines/decongestants
               15.3    Antitussives and expctorants

Appendix V

Major illness diagnosis

|  | ICD-9 code |
|---|---|
| Neoplasm (any site, any type) | 140-239 |
| Ischaemic heart disease (any form) | 410-414 |
| Pulmonary heart disease | 415-417 |
| Heart Failure | 428 |
| Cerebrovascular disease | 430-438 |
| Chronic obstructive pulmonary disease | 490-496 |
| Non infectious enteritis and colitis | 555-558 |
| Nephritis, nephrotic syndrome and nephrosis | 580-589 |
| Normal delivery and other indication for care...... | 650-659 |
| Injury and Poisoning | 800-999 |
| Suicide risk | 300.9 |
| Attempted suicide- by drug | E9502-E952 |

## Appendix VI

## Major procedures

Essentially these will be considered as any surgical procedure

CPT code 10040-69979

## Minor procedures

These are multiple screening tests and drug screening

CPT codes 80002-80103

**Claims**

1. A computer-implemented method for generating a model to identify at risk patients diagnosed with depression, information about patients existing in a claims database, said method comprising the steps of:

   processing, based on predetermined criteria, the patient information in the claims database to extract claims information for a group of depression patients;
   defining, using the information available in the claims database, a set of events relevant to depression;
   creating, using the extracted claims information and the defined events, files containing event level information;
   defining a time window for providing a timeframe from which to judge whether events should be considered in subsequent processing;
   defining a set of variables as potential predictors;
   processing the event level information, using the time window and the set of variables, to generate an analysis file; and
   performing statistical analysis on the analysis file to generate a prediction model for use in identifying at risk patients diagnosed with depression, said prediction model being a function of a subset of the set of variables.

2. A computer-implemented method for identifying at risk patients diagnosed with depression, information about patients existing in a claims database, said method comprising the steps of:

   processing, based on predetermined criteria, the patient information in the claims database to find and extract claims information for a group of depression patients;
   defining, using the information available in the claims database, a set of events relevant to depression;
   processing the extracted claims information and the defined events to create files containing event level information;
   defining a time window for providing a timeframe from which to judge whether events should be considered in subsequent processing;

defining a set of variables as potential predictors;

processing the event level information, using the time window and the set of variables, to generate an analysis file;

performing statistical analysis on the analysis file to generate a prediction model, said prediction model being a function of a subset of the set of variables; and

applying the prediction model to a processed claims database to identify and output a file listing the likelihood of each patient having an adverse health outcome.

3. The computer-implemented method of claim 1, wherein the step of processing extracts patients having been diagnosed with depression or prescribed an anti-depressant drug.

4. The computer-implemented method of claim 1, wherein the step of defining a set of variables includes defining both dependent and independent variables and a hospital (HL) indicator is defined as a dependent variable, where independent variables are representative of predictors and the dependent variable is representative of a adverse health outcome.

5. The computer-implemented method of claim 1, wherein the step of defining a set of variables includes defining both dependent and independent variables and a high cost indicator is defined as a dependent variable, where independent variables are representative of predictors and the dependent variable is representative of a adverse health outcome.

6. The computer-implemented method of claim 1, wherein the step of defining a set of variables includes defining both dependent and independent variables, substantially all of the data elements from the claims information as well as at least one combination of data elements are used as independent variables.

7. The computer-implemented method of claim 1, wherein the step of performing statistical analysis includes performing logistic regression.

8. An apparatus for generating a model to identify at risk patients with depression, information about patients existing in a claims database, said apparatus comprising:

means for processing, using predetermined criteria, the patient information in the claims database to find and extract claims information for a group of depression patients;

a predetermined set of events, derived from the claims information, said events being relevant to depression;

means, using the extracted claim information and set of events, for creating files of event level information;

a predetermined time window for providing a timeframe from which to judge whether events should be considered in subsequent processing;

a predetermined set of variables representing potential predictors;

means, using the time window and the set of variables, for processing the event level information to generate an analysis file; and

means for performing statistical analysis on the analysis file to generate a prediction model used for identifying at risk patients diagnosed with depression, said prediction model being a function of a subset of the set of variables.

9. The apparatus of claim 8, further comprising:

means for applying the prediction model to a processed claims database to identify and output a likelihood for each patient of having an adverse health outcome.

10. A computer-readable medium containing a program for generating a model to identify at risk patients diagnosed with depression from a claims database which contains information about patients, said program on said medium comprising:

means for causing a computer to process, based on predetermined criteria, the patient information in the claims database to extract claims information for a group of depression patients;

means for causing the computer to input a set of predetermined events relevant to depression;

means for causing the computer to create, using the extracted claims information and the defined events, files containing event level information;

means for causing the computer to establish a time window for providing a timeframe from which to judge whether events should be considered in subsequent processing;

means for causing the computer to input a set of predetermined variables representative of potential predictors;

means for causing the computer to process the event level information, using the time window and the input set of variables, to generate an analysis file; and

means for causing the computer to perform statistical analysis on the analysis file to generate a prediction model used for identifying at risk patients diagnosed with depression, said prediction model being a function of a subset of the set of variables.

Claims Information

Store Raw Info In Database ~110

Pre-Process Raw Info ~112

**FIG. 1A**

Store in Research Database ~114

Extract CHF Patients ~116

Quality Check (optional) ~118

OK ? — N

Y

Convert Claims Level Data to Event Level Data ~120

Process Event Level Files into Analysis Files ~122 ← timeframe ← variables

Process Analysis File Using Stat Techinques ~124

Prediction Model

```
┌─────────────────────────────┐  ⌒ 130
│      Update Claims Info      │
└─────────────────────────────┘
               │
               ▼
┌─────────────────────────────┐  ～ 132
│    Apply Prediction Model    │
└─────────────────────────────┘
               │
               ▼
┌─────────────────────────────┐  ～ 134
│  Output/Display Subgroups at │
│  High Risk                   │
└─────────────────────────────┘
```

# FIG. 1B

EP 0 789 307 A1

# Resources

FIGURE 2

**210**

## Rx

Name
date prescribed
Drug code
Amt dispensed
Physician name
Amt claimed
Amt R-reimbursed
......

**212**

## Doctor

Name
Physician
diagnosis - ICD-9
Procedures
Date
Amt Claimed
......

**214**

## Hospital

Name
Hospital - site code
Physician
Diagnosis
Date
Procedures
length of stay
......

23

EP 0 789 307 A1

# Research Data Base (RDB)

FIGURE 3

**SAS format**

Rx                           DR                           HL

| ID, date drug... | ID, ICD, date... | ID, ICD, hosp... |

claim 1

.

.

claim x

| | Event | Event date | Number | cost | Catagory indicator |
|---|---|---|---|---|---|
| 1 | Hospitalization for depression | date | LOS | $ | severity |
| 2 | Emergency room for depression | date | blank | $ | blank |
| 3 | Doctor (outpatient) visit for depression | date | blank | $ | specialist |
| 4 | Prescription for SSRI | date | days supply | $ | therapy class |
| 5 | Prescription for TCA | date | days supply | $ | therapy class |
| 6 | Prescription for other neuroactive drug | date | days supply | $ | sub-class |
| 7 | Procedure for depression | date | blank | $ | sub-class |
| 8 | Hospitalization not for depression | date | LOS | $ | severity |
| 9 | Emergency Room not for depression | date of first | number in month | $ | |
| 10 | Doctor (outpatient) visit not for depression | date of first | number in month | $ | |
| 11 | Prescription for possibly related drugs | date of first | number in month | $ | |
| 12 | Prescription for non-depression drugs | date of first | number in month | $ | |
| 13 | Procedure not for depression | date of first | number in month | $ | Severity indicator |

# FIGURE 4

EP 0 789 307 A1

# Analysis file

Variables - created from claims data elements.

$X_1$ $X_2$ $\cdot$ $\cdot$ $\cdot$ $X_n$

Member 1

Member x

FIGURE 5

EP 0 789 307 A1

# Model - Scheme 1

Hospital vs. Hospital

case definition

**Time**

-12 months      -6 months      0      +6 months

$$\text{event} \ x \ \frac{1}{e^{\text{-time}}}$$

FIGURE 6 A

EP 0 789 307 A1

# Model - Scheme 2

case definition

Last Hospital

Time

-12 months        -6 months        0    3 months

event x $\dfrac{1}{e^{-time}}$

FIGURE 6B

# Multiple Logistic model
## Hospitalization, scheme 1, 6/12, Rx + Dx

| Variable | parameter estimate | Odds ratio (CI) | P (chi square) |
| --- | --- | --- | --- |
| Intercept | -4.6010 | 0.010 (.006 - .016) | $1.0 \times 10^{00}$ |
| Depression Hosp | 1.2401 | 3.456 (2.26 -5.286) | $1.0 \times 10^{-8}$ |
| Psychiatric Hosp | 1.3942 | 4.032 (1.99 - 8.155) | $1.0 \times 10^{-4}$ |
| ER (non-dep) | 0.5719 | 1.772 (1.33 - 2.36) | $9.0 \times 10^{-5}$ |
| Depression Proc | 0.0627 | 1.065 (1.03 - 1.09) | $1.0 \times 10^{-5}$ |
| Number Gen Drs | 1.0651 | 2.901 (1.74 - 4.85) | $5.0 \times 10^{-5}$ |
| *Female* | *-0.7037* | *0.495 (0.326 - 0.75)* | *$9.0 \times 10^{-4}$* |

FIGURE 7A

# Multiple logistic regression
# High cost, scheme 1, 6/12, Rx + Dx

| Variable | parameter estimate | Odds ratio (CI) | P (chi square) |
|---|---|---|---|
| Intercept | -2.3632 | 0.090 (.037 - .237) | $5.0 \times 10^7$ |
| Sedative/anxio. | 0.1100 | 1.116 (1.092 -1.141) | $1.0 \times 10^{00}$ |
| OPD Psychiatrist | 0.083 | 1.087 (1.044 - 1.131) | $5.0 \times 10^{-5}$ |
| SSRI | 0.1955 | 1.216 (1.165 - 1.270) | $1.0 \times 10^{00}$ |
| Psychotherapy | 0.0437 | 1.045 (1.012 - 1.079) | $7.6 \times 10^{-3}$ |
| Number Gen Dr | 0.9159 | 2.499 (1.87 - 3.34) | $6.0 \times 10^{-10}$ |
| *Female* | *-0.4695* | *0.625 (0.476 - 0.822)* | *$8.0 \times 10^{-4}$* |

FIGURE 7 B

# EP 0 789 307 A1

**European Patent Office**

## EUROPEAN SEARCH REPORT

Application Number

EP 97 30 0600

### DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | US 5 486 999 A (MEBANE ANDREW H) 23 January 1996 <br> * column 6, line 1 - column 10, line 15; table 1 * | 1,2,8,10 | G06F17/00 |
| A | DATABASE MEDLINE <br> US NATIONAL LIBRARY OF MEDICINE (NLM), BETHESDA, MD, US <br> Acc. No. 07585023, <br> XP002030494 <br> * abstract * <br> & AMERICAN JOURNAL OF PSYCHIATRY, <br> vol. 150, no. 8, August 1993, USA, <br> pages 1139-1148, <br> KENDLER K.S.: "The Prediction of Major Depression in Women: Toward an Integrated Etiological Model" | 1,2,8,10 | |
| A | US 5 301 105 A (CUMMINGS JR DESMOND D) 5 April 1994 <br> * abstract; figure 1 * <br> * column 4, line 40 - column 5, line 8 * <br> * column 5, line 39 - column 6, line 12 * | 1,2,8,10 | TECHNICAL FIELDS SEARCHED (Int.Cl.6) <br><br> G06F |
| A | WO 94 04072 A (BRILL PETER L) 3 March 1994 <br> * page 5, line 23 - page 6, line 15; figures 6,7 * <br> * page 18, line 22 - page 20, line 3 * | 1,2,8,10 | |
| A | METHODS OF INFORMATION IN MEDICINE, <br> vol. 19, no. 1, 1980, <br> pages 37-41, XP000671550 <br> WOOLSON R.F. ET AL.: "Data Management in an Epidemiological Study: Experiences from the Iowa 500 field Follow-up and Family Study" | 1,2,8,10 | |

The present search report has been drawn up for all claims

| Place of search | Date of completion of the search | Examiner |
|---|---|---|
| BERLIN | 30 April 1997 | Deane, E |